# EUROPEAN PATENT SPECIFICATION

(11) **EP 1 332 742 B1**
(45) Date of publication and mention of the grant of the patent: **18.06.2008**
(21) Application number: 02002562.3
(22) Date of filing: 04.02.2002
(51) Int. Cl.: A61F 13/15

(54) **Sanitary napkin having multiple longitudinal hinges**
Damenbinde mit mehreren in Längsrichtung angeordneten Knickbereichen
Serviette hygiénique avec des régions flexibles longitudinales

(43) Date of publication of application: 06.08.2003
(73) Proprietor: McNEIL-PPC, Inc., Skillman, NJ 08558 (US)
(72) Inventor: Blanchard, Stephen John, North Brunswick, NJ 08902 (US); Briggs, Amelie, Lawrenceville, NJ 08648 (US); Manusco, Michele, Somerville, NJ 08876 (US); Wysocki, Theresa, Flemington, NJ 08822 (US)
(74) Representative: Metten, Karl-Heinz

(56) References cited:
- EP-A- 0 852 938
- EP-A- 0 904 755
- WO-A-00/19956
- WO-A-01/26595
- WO-A-02/07663
- US-A- 4 758 240
- US-A- 5 514 104

## Description

### FIELD OF THE INVENTION

This invention relates to protective absorbent products used by women for feminine hygiene, and more particularly to improved sanitary protection products such as sanitary napkins, panty liners and incontinence products that, in use, fit closely and conform to the body, resist transverse bunching and reduce or prevent leakage of liquid from the edges.

### BACKGROUND OF THE INVENTION

Sanitary napkins are commonly used by women for feminine hygiene to absorb body liquids, such as menstrual liquids and urine. It is important that these products prevent such liquids from escaping the confines of the absorbent materials and edges of such products and thereby staining the wearer's undergarments and outer clothing. Current product designs attempt to provide coverage of the body without being bunched, twisted, folded or otherwise having their absorbent regions reduced in size and removed from contact with the user's body. Many of these products are adapted to fit close to the body to prevent liquid from running along the body before it is absorbed. Such sanitary napkins may optionally have flexible flaps (or wings) extending from the side edges of the main body of the napkin that are adapted to be folded over the edges of a crotch portion of the undergarment. Alternative flap designs are attached to the napkin on a garment faceable surface, inward of the longitudinal edges of the napkin.

Various attempts have been made to prevent napkins from bending and bunching by reducing the size of the central absorbent portion of the napkin or by providing an embossed arcuate channel along the longitudinal side edges. Several inventions describe the use of centrally located channels, pleats or slits that generally follow the longitudinal dimension of the napkin, some of which also reduce the width of the product or promote reduction of its width in use. For example deeply densified end channels have been impressed through the cover and core of a napkin adjacent the lateral edges to provide resistance to bending and bunching. These attempts have been focused on achieving a particular shape either before or during the use of the product such as a raised center or a "W" shape. However, the designed shapes of these products do not fit the entire range of user anatomies. While a particular napkin may fit one user, the same napkin may deform in use by another user and result in product failure or discomfort.

### SUMMARY OF THE INVENTION

It is an object of the present invention to provide a disposable absorbent product for use in feminine hygiene that is capable of adapting to and closely fitting the perineal area of the wearer.

In accordance with the present invention, there has been provided a sanitary napkin for use by a woman for feminine hygiene and adapted to be worn in an undergarment, comprising a main body portion having a liquid permeable body faceable cover layer, a liquid impermeable garment faceable barrier layer, an absorbent element therebetween, the main body portion further having first and second longitudinal edges opposite each other defining a width dimension, a central longitudinal axis parallel to the longitudinal edges, first and second transverse edges opposite each other defining a length dimension;
a central region having first and second distal ends opposite each other defining a length that is sufficient to cover the woman's labia majora in use;
a first end region, extending from the first distal end of the central region to the first transverse edge and being adapted to cover at least a portion of the woman's mons pubis in use;
a second end region, extending from the second distal end of the central region to the second transverse edge and being adapted to cover at least a portion of the woman's posterior perineum in use;
a densified end channel in at least one of the first and second end regions; and
at least two longitudinally extending hinges located substantially within the center region adjacent each longitudinal edge and said densified end channel is spaced apart from said hinges by at least 3 mm each hinge adapted to provide a longitudinally extending preferential bending axis, said hinges being spaced apart along at least a portion of their length, wherein at least one hinge has a radius of curvature and an adjacent hinge is substantially straight.

a central region having first and second distal ends opposite each other defining a length that is sufficient to cover the woman's labia majora in use;
a first end region, extending from the first distal end of the central region to the first transverse edge and being adapted to cover at least a portion of the woman's mons pubis in use;
a second end region, extending from the second distal end of the central region to the second transverse edge and being adapted to cover at least a portion of the woman's posterior perineum in use; and
at least two longitudinally extending hinges located substantially within the center region adjacent each longitudinal edge, each hinge adapted to provide a longitudinally extending preferential bending axis, said hinges being spaced apart along at least a portion of their length, wherein each of said hinges have a radius of curvature and wherein one hinge has a radius of curvature greater than an adjacent hinge and wherein the sanitary napkin preferentially bends along the hinges.

### BRIEF DESCRIPTION OF THE DRAWINGS

Fig. **1** is a top plan view of a sanitary napkin of this invention;
Fig. **2** is a cross sectional view taken through axis 2-2 of Fig. **1****,** wherein the stiffened element is an densified end channel
Fig. **3** is a top plan view of an alternative embodiment of a sanitary napkin of this invention;
Fig. **4** is a top plan view of an alternative embodiment of a sanitary napkin of this invention;
Fig. **5** is a top plan view of an alternative embodiment of a sanitary napkin of this invention; and
Fig. **6** is a top plan view of an alternative embodiment of a sanitary napkin of this invention.

### DETAILED DESCRIPTION OF THE INVENTION

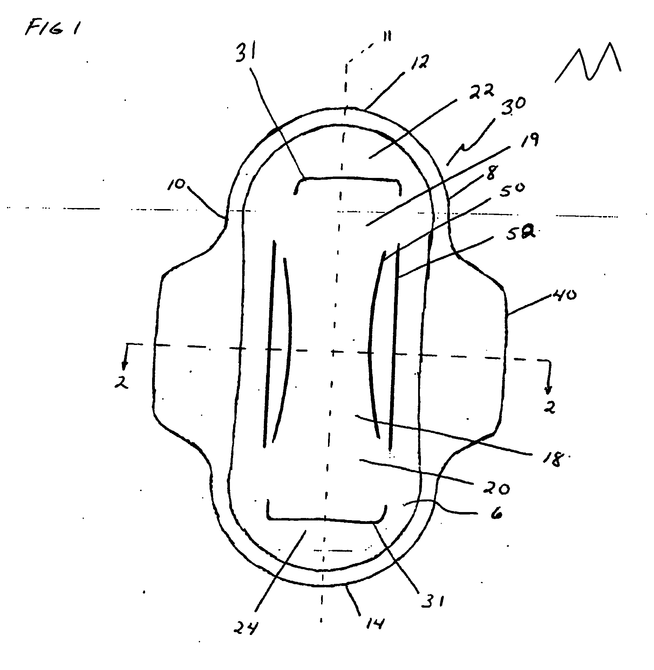

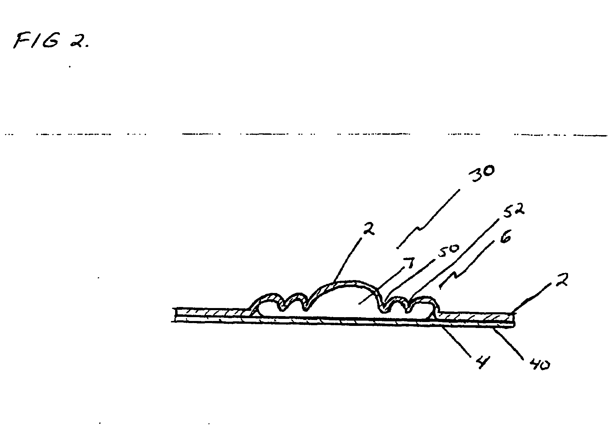

The present invention is directed to a sanitary napkin for feminine hygiene that is adapted to be worn in a crotch portion of a user's undergarment. Referring now to the drawings in detail, wherein like numerals indicate the same elements throughout the views, Fig. **1** is a top plan view of a sanitary napkin **30** and Fig **2** is a cross-sectional view of the sanitary napkin in Fig. **1** shows a main body portion **6** comprising a liquid permeable body faceable cover layer **2**, a liquid impermeable garment faceable barrier layer **4**, an absorbent element **7** between the liquid permeable body faceable cover layer **2** and the liquid impermeable garment faceable barrier layer **4**. The main body portion **6** has first and second longitudinal edges **8, 10** opposite each other defining therebetween a width dimension, a central longitudinal axis **11** parallel to longitudinal edges **8, 10,** first and second transverse edges **12, 14** opposite each other defining therebetween a length dimension and a thickness dimension (or caliper).

The main body portion is made up of three regions, a central region **18,** a first end region **22** and a second end region **24** opposite the first end region **22.** The center region **18** has a length which is sufficient to cover the woman's vestibule and labia major in use. The central region **18** has longitudinal edges which are coincident with longitudinal edges **8, 10** of main body portion **6** and first and second distal ends **19, 20** opposite each other. The first and second distal ends **19, 20** are located intermediate the first and second transverse edges **12, 14** of the main body portion **6** and are generally located inward of each respective transverse edge about one third of the length of the main body portion.

The first end region **22** is intermediate the first distal end **19** of central region **18** and the first transverse edge **12.** The first end region **22** is adapted to cover at least a portion of the woman's mons pubis in use. The second end region **24** is intermediate the second distal end **20** of central region **18** and the second transverse edge **14.** The second end region **24** is adapted to cover at least a portion of the woman's posterior perineum in use. Thus, the central region **18** is adapted to be substantially vertically aligned with a user's vaginal opening, the first end region **22** is adapted to be worn in an anterior region of a user's groin region and the second end region **24** adapted to be worn in a posterior region of a user's groin region. The central region **18** defines an area that is sufficient to cover at least the user's vestibule and labia majora in use.

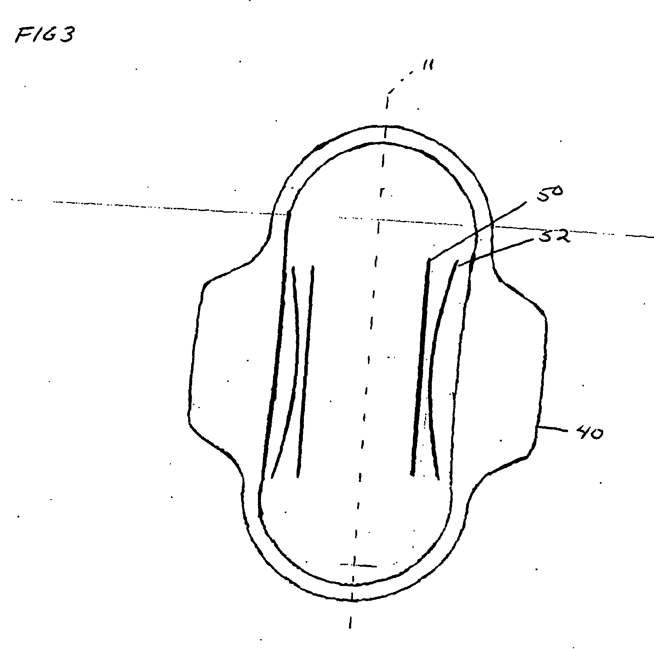

In accordance with a first embodiment of the present invention, the sanitary napkin **30** comprises a main body portion **6** having a liquid permeable body faceable cover layer **2**, a liquid impermeable garment faceable barrier layer **4**, an absorbent element **7** therebetween, the main body portion **6** further having first and second longitudinal edges **8, 10** opposite each other defining a width dimension, a central longitudinal axis **11** parallel to the longitudinal edges **8, 10,** first and second transverse edges **12, 14** opposite each other defining a length dimension; a central region **18** having first and second distal ends **19, 20** opposite each other defining a length that is sufficient to cover the woman's labia majora in use; a first end region **22,** extending from the first distal end **19** of the central region to the first transverse edge **12** and being adapted to cover at least a portion of the woman's mons pubis in use; a second end region **24,** extending from the second distal end **20** of the central region **18** to the second transverse edge **14** and being adapted to cover at least a portion of the woman's posterior perineum in use; and at least two longitudinally extending hinges **50, 52** located substantially within the center region **18** adjacent each respective longitudinal edge **8, 10,** each hinge adapted to provide a longitudinally extending preferential bending axis, said hinges being spaced apart along at least a portion of their length, wherein at least one hinge has a radius of curvature and an adjacent hinge is substantially straight. As shown in Fig **1** a first hinge **50** has a radius of curvature and is closer to the longitudinal centerline **11** than the second hinge **52** that is substantially straight. Alternatively, as shown in Fig. **3**, the first hinge **50** is substantially straight and is closer to the longitudinal centerline **11** than the second hinge **52** that has a radius of curvature.

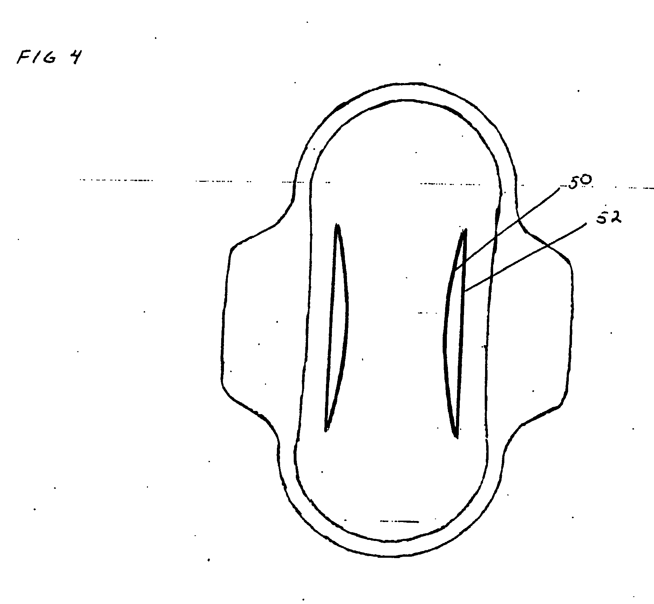

The embodiment shown in Figure **4** is similar to the embodiment in Figure **1**, and illustrates that the first hinge **50** is separated from the second hinge **52** along a substantial portion of their respective lengths but are co-terminous and contact one another at their respective distal ends.

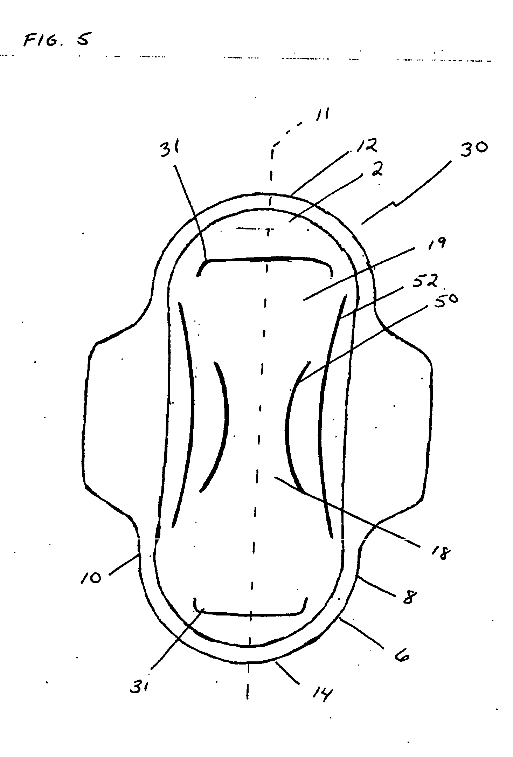

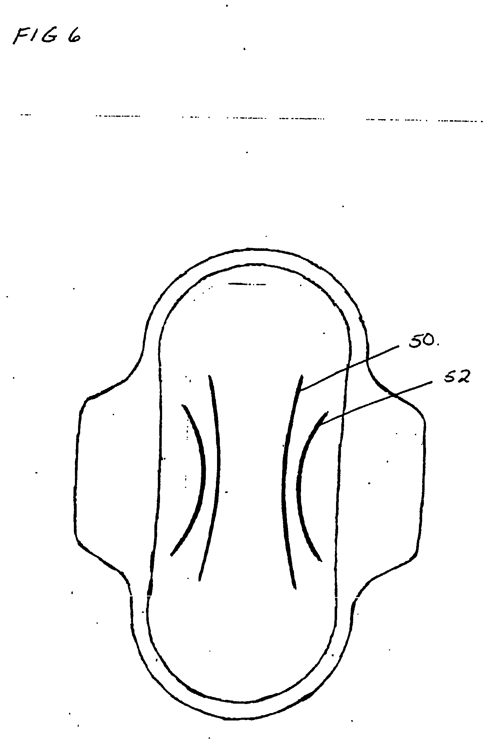

In accordance with another embodiment of the present invention as shown in Figs. **5** and **6**, the sanitary napkin **30** comprises a main body portion **6** having a liquid permeable body faceable cover layer **4**, a liquid impermeable garment faceable barrier layer (not shown), an absorbent element (not shown) therebetween, the main body portion **6** further having first and second longitudinal edges **8, 10** opposite each other defining a width dimension, a central longitudinal axis **11** parallel to the longitudinal edges **8, 10,** first and second transverse edges **12, 14** opposite each other defining a length dimension; a central region **18** having first and second distal ends **19, 20** opposite each other defining a length that is sufficient to cover the woman's labia majora in use; a first end region **22,** extending from the first distal end **19** of the central region to the first transverse edge **12** and being adapted to cover at least a portion of the woman's mons pubis in use; a second end region **24,** extending from the second distal end **20** of the central region **18** to the second transverse edge **14** and being adapted to cover at least a portion of the woman's posterior perineum in use; and at least two longitudinally extending hinges **50, 52** located substantially within the center region **18** adjacent each respective longitudinal edge **8, 10,** each hinge adapted to provide a longitudinally extending preferential bending axis, said hinges being spaced apart along at least a portion of their length, each hinge having a radius of curvature and wherein one hinge has a radius of curvature greater than an adjacent hinge and wherein the sanitary napkin preferentially bends along the hinge.

As shown if Fig. **5**, the first hinge **50** has a lower radius of curvature and is closer to the longitudinal centerline **11** than the second hinge **52** that has a greater radius of curvature relative to the first hinge **50.** Alternatively, as shown in Fig. **6**, the first hinge **50** has a greater radius of curvature and is closer to the longitudinal centerline **11** than the second hinge **52** that has a lower radius of curvature relative to the first hinge **50.**

In any of the foregoing embodiments, it has been found that by providing a sanitary napkin with at least two substantially centrally located longitudinal hinges adjacent each longitudinal side edge of the main body, wherein one hinge has a greater radius of curvature than the adjacent hinge provides an optimum fit to a wide variety of wearers' anatomies. That is the multiple longitudinal hinges create a sanitary napkin that has multiple modes of controlled deformation that enables the napkin to conform to the entire range of user anatomies. Moreover, the multiple longitudinal hinges control bunching rather than prevent bunching to create greater comfort, fit to the anatomy and discretion.

It is thus considered an important feature of the present invention that one hinge has a greater radius of curvature than the adjacent hinge. It should be noted in this regard that for purposes of this invention, one hinge may be substantially straight provided that the adjacent hinge has a radius of curvature. The combination of multiple hinges with differences in curvature provides a controlled deformation in an enhanced manner. A hinge having a lower radius of curvature (less straight) has more lateral stability than a hinge having a higher radius of curvature (more straight) since it has more lateral components and can thus resist transverse compression. The hinge with a higher radius of curvature will have a greater tendency to bend relative to a hinge with a lower radius of curvature. Thus the hinge with the lower radius of curvature has a greater resistance to bunching and will maintain the napkin is a flatter (i.e. more planar) configuration in use. The combination of these two hinge types along each longitudinal side of the main body enables the main body to conform to a wide range of user anatomies.

Hinges **50, 52** may include any material in an amount sufficient to impart additional structural rigidity relative to adjacent regions. Examples of suitable material to form the hinges **50, 52** comprise a sphagnum-moss containing insert, a densified channel, strips of polymeric foam, and the like and combinations thereof. In a preferred embodiment, each hinge **50, 52** is created by compressing, embossing or scoring one or more layers of the absorbent structure in an amount sufficient to create a longitudinally extending preferential bending line. When the hinges **50, 52** comprise a densified channels, they are preferably located between the central longitudinal axis and the longitudinal edge. Densified side channels may be formed in the main body by heating and compressing the cover layer/absorbent element/barrier layer assembly in selected areas to form densified channels similar to those shown in Figs **1-6****.** The density of the channels is at least two times the density of the adjacent non-densified regions and is preferably from two to ten times the density of the adjacent regions of the absorbent structure **7**. The density of the channels is preferably at least 0.5 g/cc. The hinges **50, 52** are adapted to maintain the center region of the main body portion in a relatively flat profile along the longitudinal axis and resists bending transverse to that axis. A center region having a hinges **50, 52** has been found to effectively conform to the body in that region, resist asymmetrical deformation due to the application of laterally compressive forces and thereby prevents leakage of liquid from the main body portion. That is, since the hinges effectively control deformation, when subjected to laterally compressive forces of a user's thighs, the sanitary napkin will deform symmetrically about the longitudinal centerline rather than deforming randomly across its width.

In an optional embodiment, the sanitary napkin may be provided with one or more densified end channels **31** in a transverse end region **22** and/or **24** on the napkin. The densified end channels **31** are preferably located intermediate the distal ends **19, 20** of central region **18** and the hinges **50, 52.** The densified end channels **31** are preferably located inward of the transverse edges **12, 14** and longitudinal edges **8, 10** of end regions **22, 24** of the main body portion **6**, and are preferably approximately between 3 mm to 15 mm inward of the transverse edges **12, 14** of the respective end regions **22, 24.**

The densified end channels **31** extend transversely across end regions **22,** or **24** respectively, generally perpendicular to central longitudinal axis **11** of main body portion **6**, at least at its intersection with that axis, and centrally occupies at least 50% of the width of the main body portion. The densified end channels **31** resist transverse bunching in use relative to the non-stiffened region. As used herein, the terminology "resists transverse bunching" refers to the ability of the densified end channels **31** to resist deformation or bending when subjected to the application of laterally compressive forces. The densified end channels **31** provide an axis of bending which is coincident with the transverse axis of napkin **30** and resists bending and compression along this transverse axis **11** of napkin **30.** The densified end channels **31** may also act as a barrier to liquid wicking and guide the liquid so that it is retained within the confines of the main body portion. Thus, it is another benefit that the densified end channels 32 confine liquid within main body portion **6.** The densified end channels **31** thereby provides a preferential bending axis that is generally perpendicular to the longitudinal axis of the napkin, a resistance to bending and compression perpendicular to the longitudinal axis and will curve in use to fit and conform to the user's body. The densified end channels **31** are spaced apart from the hinges **50, 52** by at least 3 mm, preferably by at least 5 mm and most preferably by at least 10 mm. The spacing apart of the densified end channels **31** from the hinges **50, 52** in use, enables the sanitary napkin to preferentially bend transversely to the longitudinal axis of the main body portion **6** and to closely fit the body along the length of napkin **30.**

The densified end channels **31** provides a transverse bending means to create a lateral axis of flexibility remote from the hinges **50, 52** that allows the napkin to flex longitudinally while providing rigidity against transverse compression and bunching in use. In a preferred embodiment of the invention, the densified end channels 32 are relatively bendable in a direction parallel to their length, and thus form a hinge. The densified end channels **31** stiffen the napkin in directions perpendicular to the channels along their entire length. Therefore, channels that are aligned transversely in napkin **30** will resist bunching in a transverse direction and channels that have a longitudinally oriented component will resist compression in the longitudinal direction of napkin **30.** Thus, densified end channels 32 , and their adjacent hinges **50, 52,** cooperate to bend, conform and closely fit the body, to resist transverse compression and bunching, and at times longitudinal compression and prevent-leakage of liquid from napkin's **30** edges. Densified end channels may be impressed into the napkin **30,** either from the body faceable side or from the garment faceable side of the absorbent element. Thus, densified end channels may be impressed into napkin **30** either from the body faceable cover layer **2** or from the garment faceable barrier layer **4** through the any part of the thickness of main body portion **6**.

The densified end channels **31** may have any shape or configuration and may comprise continuous segments, discontinuous segments, straight segments, curved segments or combination thereof. It is preferred that the densified end channels **31** be continuous. If the densified end channels **31** are discontinuous, it is preferred that the discontinuous segments be spaced apart from each other by no more than 0.25 inches in order to maintain good liquid barrier properties and deformation and bunching resistance for the main body portion.

In a most preferred embodiment of the invention, the densified end channels **32** have at least one perpendicular component that is generally orthogonal to the lateral component and generally parallel to the central longitudinal axis of the absorbent product. Preferably the perpendicular component is closer to the longitudinal edge than it is to the central longitudinal axis. The perpendicular component need not be strictly orthogonal to the lateral component. It may also be at an acute or obtuse angle to the lateral component, and may extend towards the transverse edge and/or towards the longitudinal center line.

The center region of the absorbent product of this invention thus has hinges **50, 52** that are spaced apart from the densified end channels. It is important that the hinges **50, 52** be located in the center region of the sanitary napkin and spaced apart from the densified end channels so as not to compromise the conformability and body fit provided by the densified end channels. The hinges **50, 52** maintains the center region of the main body portion **6** in a relatively flat profile along the longitudinal axis, in order to best cover and closely fit the body in that region and to promote controlled deformation in use, and thereby prevent leakage of liquid from the main body portion **6**. Accordingly, when the hinges **50, 52** comprise densified channels, they are preferably located between the central longitudinal axis and the longitudinal edges and the hinges **50, 52** in the central region are spaced apart from the densified end channels in the end region(s).

The sanitary napkins **30** of this invention may be provided with a means for attaching it to the undergarment in use. Such attachment means include adhesive which may be protected by a strip of release paper until use, or by mechanical attachments such as a hook and loop assembly, clasp assembly or by combinations thereof. The strip of release paper may be eliminated if the sanitary napkins of this invention are packaged in a protective outer wrapper that has a napkin faceable surface that is of itself releasable from adhesive by a coating of a release substance such as silicone or fluorocarbon or by being physically altered, such as by embossing, to reduce its contact with the adhesive.

A suitable body faceable cover layer **2** may be comprised of at least a single layer or combinations of apertured polymeric film or foam or fabrics such as woven fabrics, knits and nonwoven fabrics. Suitable nonwoven fabrics include spunbond, meltblown, needle-punched, thermobonded, chemical binder bonded, powder bonded, solvent bonded and hydro-entangled nonwoven fabrics. Apertured polymeric films may be such as those whose surfaces are flat or embossed such as a matte finish having a smooth feel. The apertures may be two-dimensional, being essentially restricted to the plane of the film, or three-dimensional, where the apertures have side walls that extend either above or below the plane of the film. The surfaces of body faceable cover layers may be hydrophobic, hydrophilic, or one surface may be hydrophobic and the other hydrophilic or the surface may have a gradient of hydrophobicity to hydrophilicity from one surface to the other. The body faceable cover layer **2** of the sanitary napkin **30** of this invention generally covers all of the upper surface of the absorbent element **7** and generally extends beyond the sides of the absorbent element. The body faceable cover layer **2** may be adhesively affixed or otherwise adhered to the surface of the absorbent element.

The garment faceable barrier layer **4** generally covers the entire garment faceable surface of the absorbent element and generally extends beyond the sides of the absorbent element **7**. The garment faceable barrier layer **4** may be adhesively affixed to the surface of the absorbent element **7**. The garment faceable barrier layer **4** is generally adhered to the body faceable cover layer **2** in a flange seal extending along the peripheral edge margins of the longitudinal and transverse sides of the main body portion **6** to fully enclose the absorbent element **7**. The impermeable garment faceable barrier layer **4** may be formed of any flexible material that prevents the transfer through it of liquid but does not necessarily prevent the passages of gases. Commonly used materials are polyethylene or polypropylene films.
Other materials that may be used as impermeable barriers may be chosen from films of polyesters, polyamides, ethylene vinyl acetate, polyvinyl chloride, polyvinylidene chloride, cellophane, nitrocellulose and cellulose acetate. Co-extruded and laminated combinations of the foregoing, wherein such combinations are permitted by the chemical and physical properties of the film, may be used. Fabrics whose surfaces have been made repellent or whose pores are small by virtue of close packing of fibers, or whose pores have been reduced in size by closing off large liquid admitting pores, may also be used alone, or together with breathable films, as breathable barriers.

The absorbent element **7** may comprise a single layer absorbent core and may optionally further include a liquid acquisition layer (not shown) which is commonly referred to as a transfer layer, that is capable of rapidly acquiring liquid and holding it until a slower absorbing absorbent core is capable of absorbing the liquid. The absorbent element comprises absorbent materials that accept, transfer, distribute, store and retain liquid as well as prevent liquid from exiting the absorbent product.

The absorbent core is generally a single layer absorbent material such as wood pulp fluff, preferably comprises a mixture of wood pulp fluff and superabsorbent particles. The wood pulp itself may also be comprised, at least in part, of any of wet crosslinked, dry crosslinked, chemically stiffened or curly fibers. The absorbent core may further contain rayon fibers, cotton fibers, sphagnum moss, superabsorbent fibers, stabilizing components such as synthetic fibers that are capable of forming a bridging matrix or thermobondable synthetic fibers that may be fused to themselves and to the wood pulp to form a structure that is stable when wetted. The synthetic fibers may be either hydrophilic, such as rayon, or hydrophobic such as polypropylene and polyester. The synthetic fibers may be made more wettable by treatment with a wetting agent such as a surfactant, by caustic etching of fibers such as polyester, by incorporating wettable polymers such as polyethylene oxide or polyvinyl alcohol within the fiber polymer formulation, by grafting the fiber surface with wettable reactants and by exposing the fiber to corona discharge. The peripheral profile of synthetic fibers may be of any shape, e.g., round, oval, multi-lobed.

Additional absorbent materials such as sphagnum moss, in board or in compressed layer form, may function additionally as compression resisting or deformation resisting structures or to help maintain a flat or raised product profile. Absorbents in board form may be made flexible and conforming by tenderizing by means of passing the board through a corrugating or embossing process. The synthetic fibers and auxiliary absorbents may be present homogeneously throughout the absorbent core, in discrete layers or in continuous or discontinuous concentration gradients.

As discussed above, the absorbent element **7** may contain, in addition to the absorbent core, a transfer layer (not shown), which is a low density liquid accepting and liquid releasing layer, usually located between the absorbent core and the liquid permeable body faceable cover layer **2**. The transfer layer may be comprised of relatively less hydrophilic materials and structures, than is contained in the absorbent core, such as of webs of meltblown polypropylene or polyester fibers. Such webs may also contain wood pulp entrained within. Transfer layers may also be comprised of low density, highloft nonwoven webs comprised of wood pulp and synthetic fibers such as polyethylene, polypropylene, polyester, polyacrylonitrile and polyamide. Such highloft webs may be bonded with chemical binders or by thermal means such as by through-air bonding.

The main body portion **6** may be of many different shapes and sizes, depending on the requirements of the user with reference to her anatomy, menstrual flow volume and intensity, duration of wear and the part of the day or night the product is being worn. For example, longitudinal sides may be generally straight or alternatively may be somewhat curved and are preferably in a substantially dog-bone or hour-glass shape with wider end regions and a tapered central region. The end regions may or may not be symmetrical about the central region. The end regions may or may not be the same shape or size as each other. The main body portion **6** of the sanitary napkin **30** of this invention will have the following approximate dimensional ranges: a length of between 12 cm and 35 cm, a width in a range of between 35 mm and 75 mm, and a thickness in a range of between 2 mm and 10 mm, and is preferably less than 4 mm. The thickness of the main body portions of this invention may be uniform throughout the expanse of the main body portion or, for the purpose of specific fit, flexibility and absorbency requirements, the main body portion may be thicker in some regions than in others. For example, a particularly preferred thickness profile is an absorbent element that is thicker in the central region and thinner in the transverse end regions and along the longitudinal edges.

The sanitary napkins of this invention may also comprise additional components that may add to the functional, comfort and aesthetic properties of the products such as upstanding cuffs (not shown) along the longitudinal sides of the main body or flexible flaps **40** extending laterally outward from the longitudinal sides which are adapted to be folded over the edges of a crotch portion of a wearer's undergarment in use. Such flaps **40** cooperate with the densified end channels of this invention to help maintain the surface of the central region of the napkin in a flat and spread open position along the napkin's longitudinal axis. In a most preferred embodiment, the flaps **40** are attached inward of the longitudinal edges of the main body portion on a garment faceable side of the napkin. These flaps **40** not only maintain the crotch portion of the undergarment inward from the longitudinal side edges of the napkin to provide enhanced protection, but the elastic in the undergarment is substantially vertically aligned with the second longitudinal hinge **52** which creates an upward force to the napkin. This upward force creates a preferential bending axis along the second hinge **52** and enables the napkin to more easily conform to the user's body.

The flaps **40** may be formed from separate strips of material attached to the main body **6** of the napkin or, alternatively may be continuous extensions of the cover layer **2** and/or barrier layer **4.** The flaps **40** may be provided with adhesive, protected with release paper, for attachment to the undergarment. The flaps **40** may also contain additional materials to make them thick and cushioning and may also contain, separately or additionally, flexible, stretchable or elastic materials. Embodiments of cuffs and flaps such as are described herein, and which are incorporated herein in their entirety are described in the following commonly assigned U.S. Patents: US Patent No. 4,940,462 to Salerno, "Sanitary Napkin with Expandable Flaps"; US Patent No. 5,490,847 to Correa et al; "Disposable Sanitary Napkin", US Patent No. 4,900,320 to McCoy, "Sanitary Napkin with Panty Gathering Flaps".

Figs. **1** and **2** show a sanitary napkin of this invention having right and left longitudinally extending flaps **40,** each flap **40** being attached along its respective base portion to the right and left longitudinal edges **8, 10** of main body portion **6**, respectively, such that a freely extending distal end extends outward from the right and left longitudinal edges **8, 10** of main body portion **6**. Each flap **40** is formed from a continuous extension of the body faceable cover layer **2** and the garment faceable barrier layer **4** and which are attached to each other throughout the body of the flap **40.** The main body portion **6** of this invention also comprises end regions **22, 24,** each having a densified end channels **32,** respectively and a central region **18** having a hinges **50, 52** in the form of two densified end channels. Main body portion **6** in Fig. **1** has a substantially rectangular shape with rounded transverse edges **12, 14.** Napkin **30,** as well as flaps **40,** also comprise positioning adhesive (not shown), on their garment faceable sides, for attaching napkin **30** and flaps **40,** to an undergarment, positioning adhesive being protected until use by a release paper (not shown).

## Claims

1. A sanitary napkin for use by a woman for feminine hygiene and adapted to be worn in an undergarment, comprising a main body portion (6) having a liquid permeable body faceable cover layer (2), a liquid impermeable garment faceable barrier layer (4), an absorbent element (7) there between, the main body portion (6) further having first and second longitudinal edges (8, 10) opposite each other defining a width dimension, a central longitudinal axis (11) parallel to the longitudinal edges (8, 10), first and second transverse edges (12, 14) opposite each other defining a length dimension;
a central region (18) having first and second distal ends (19, 20) opposite each other defining a length that is sufficient to cover the woman's labia majora in use;
a first end region (22), extending from the first distal end (19) of the central region (18) to the first transverse edge (12) and being adapted to cover at least a portion of the woman's mons pubis in use;
a second end region (24), extending from the second distal end of the central region (18) to the second transverse edge (12) and being adapted to cover at least a portion of the woman's posterior perineum in use; and
a densified end channel (31) in at least one of the first and second end regions (22, 24)
**characterized in that** at least two longitudinally extending hinges (50, 52) are located within the center region (18) adjacent each longitudinal edge (8, 10) and said densified end channel is spaced apart from said hinges by at least 3 mm, each hinge (50, 52) adapted to provide a longitudinally extending preferential bending axis, said hinges (50, 52) being spaced apart along at least a portion of their length, wherein at least one hinge (50, 52) has a radius of curvature and an adjacent hinge (50, 52) is straight.

2. The sanitary napkin according to claim 1, wherein first hinge has a radius of curvature and is closer to the longitudinal centerline than the second hinge that is straight.

3. The sanitary napkin according to claim 1 wherein, the first hinge is substantially straight and is closer to the longitudinal centerline than the second hinge that has a radius of curvature.

4. The sanitary napkin according to claim 1, wherein the first hinge is separated from the second hinge along a substantial portion of their respective lengths but are co-terminous and contact one another at their respective distal ends.

5. The sanitary napkin according to claim 1, wherein said densified end channel is spaced apart from said hinges by at least 5 mm.

6. The sanitary napkin according to claim 5, wherein said densified end channel is spaced apart from said hinges by at least 10 mm.

7. A sanitary napkin for use by a woman for feminine hygiene and adapted to be worn in an undergarment, comprising a main body portion (6) having a liquid permeable body faceable cover layer (2), a liquid impermeable garment faceable barrier layer (4), an absorbent element (7) there between, the main body portion (6) further having first and second longitudinal edges (8, 10) opposite each other defining a width dimension, a central longitudinal axis (11) parallel to the longitudinal edges (8, 10), first and second transverse edges (12,14) opposite each other defining a length dimension;
a central region (18) having first and second distal ends (19, 20) opposite each other defining a length that is sufficient to cover the woman's labia majora in use;
a first end region (22), extending from the first distal end (19) of the central region (18) to the first transverse edge (12) and being adapted to cover at least a portion of the woman's mons pubis in use;
a second end region (24), extending from the second distal end of the central region (18) to the second transverse edge (12) and being adapted to cover at least a portion of the woman's posterior perineum in use; and
a densified end channel (31) in at least one of the first and second end regions (22, 24)
**characterized in that** at least two longitudinally extending hinges (50, 52) are located within the center region (18) adjacent each longitudinal edge (8, 10) and said densified end channel (31) is spaced apart from said hinges by at least 3 mm, each hinge (50, 52) adapted to provide a longitudinally extending preferential bending axis, said hinges (50, 52) being spaced apart along at least a portion of their length, wherein said at least two hinges each have a radius of curvature and wherein one hinge has a radius of curvature greater than an adjacent hinge and wherein the sanitary napkin preferentially bends along the hinge.

8. A sanitary napkin according to claim 7 wherein a first hinge has a greater radius of curvature relative to a second hinge and is closer to the longitudinal centerline than the second hinge that has a lower radius of curvature relative to the first hinge.

9. The sanitary napkin according to claim 7, wherein the first hinge has a lower radius of curvature and is closer to the longitudinal centerline than the second hinge that has a greater radius of curvature relative to the first hinge.

## Patentansprüche

1. Hygienedamenbinde zum Tragen unter Unterwäsche, umfassend einen Hauptkörperabschnitt (6) mit einer flüssigkeitsdurchlässigen, dem Körper zuwendbaren Decklage (2), einer flüssigkeitsundurchlässigen, der Kleidung zuwendbaren Sperrlage (4), ein Absorptionsteil (7) dazwischen, wobei der Hauptkörperabschnitt (6) außerdem einen ersten und einen zweiten Längsrand (8, 10), welche Längsräder sich gegenüberliegen und eine Breitenabmessung definieren, eine Mittellängsachse (11), die parallel zu den Längsrändern (8, 10) ist, und einen ersten und einen zweiten Querrand (12, 14) aufweist, welche Querränder sich gegenüberliegen und eine Längenabmessung definieren;
einen Mittelbereich (18), der ein erstes und ein zweites distales Ende (19, 20) aufweist, welche distalen Enden sich gegenüberliegen und eine Länge definieren, die ausreicht, die Schamlippen einer Frau im Gebrauch zu bedecken;
einen ersten Endbereich (22), der sich von dem distalen Ende (19) des Mittelbereichs (18) hin zu dem ersten Querrand (12) erstreckt und zum Bedecken wenigstens eines Teils des Schambergs einer Frau im Gebrauch ausgelegt ist;
einen zweiten Endbereich (24), der sich von dem zweiten distalen Ende des Mittelbereichs (18) hin zum zweiten Querrand (12) erstreckt und zum Abdecken wenigstens eins Teils des hinteren Damms einer Frau im Gebrauch ausgelegt ist; und
einen verdichteten Endkanal (31) entweder am ersten Endbereich (22) oder am zweiten Endbereich (24),
**dadurch gekennzeichnet, daß** wenigstens zwei sich in Längsrichtung erstrekkende Gelenke (50, 52) innerhalb des Mittelbereichs (18) benachbart jedem Längsrand (8, 10) angeordnet sind und der verdichtete Endkanal in einem Abstand von wenigstens 3 mm von den Gelenken angeordnet ist, wobei jedes Gelenk (50, 52) eine sich in Längsrichtung erstreckende Vorzugsbiegeachse bereitstellen kann, wobei die Gelenke (50, 52) entlang zumindest eines Teils dessen Länge in einem Abstand zueinander angeordnet sind, wobei wenigstens ein Gelenk (50, 52) einen Krümmungsradius aufweist und das benachbarte Gelenk (50, 52) geradlinig ist.

2. Hygienedamenbinde nach Anspruch 1, bei der das erste Gelenk einen Krümmungsradius aufweist und näher an der Längsmittellinie liegt als das zweite Gelenk, das geradlinig ist.

3. Hygienedamenbinde nach Anspruch 1, bei der das erste Gelenk im wesentlichen geradlinig ist und der Längsmittellinie näher liegt, als das zweite Gelenk, das einen Krümmungsradius aufweist.

4. Hygienedamenbinde nach Anspruch 1, bei der das erste Gelenk von dem zweiten Gelenk längs eines wesentlichen Abschnitts ihrer jeweiligen Länge ist, wobei sie allerdings an deren jeweiligen distalen Enden aneinander grenzen und einander berühren.

5. Hygienedamenbinde nach Anspruch 1, bei der der verdichtete Endkanal in einem Abstand von wenigstens 5 mm von den Gelenken angeordnet ist.

6. Hygienedamenbinde nach Anspruch 5, bei der der verdichtete Endkanal in einem Abstand von wenigstens 10 mm von Gelenken angeordnet ist.

7. Damenhygienegelenk zum Tragen unter Unterwäsche, umfassend einen Hauptkörperabschnitt 6 mit einer flüssigkeitsdurchlässigen, dem Körper zuwendbaren Decklage 2, einer flüssigkeitsundurchlässigen, der Kleidung zuwendbaren Sperrlage (4), einem Absorptionsteil (7) dazwischen, wobei der Hauptkörperabschnitt (6) außerdem einen ersten und einen zweiten Längsrand (8, 10), welche Längsränder sich gegenüberliegen und eine Breitenabmessung definieren, eine Längsmittelachse (11), die parallel zu den Längsrändern (8, 10) liegt, sowie einen ersten und einen zweiten Querrand (12, 14), welche Querränder sich gegenüber liegen und eine Längenabmessung definieren;
einen Mittelbereich (18) mit einem ersten und einem zweiten distalen Ende (19, 20), welche distalen Enden sich gegenüberliegen und eine Länge definieren, die ausreicht, die Schamlippen einer Frau im Gebrauch zu bedecken;
einen ersten Endbereich (22), der sich von dem ersten distalen Ende (19) des zentralen Bereichs (18) hin zu dem ersten Querrand (12) erstreckt und zum Abdecken wenigstens eines Teils des Schambergs einer Frau im Gebrauch ausgelegt ist;
einen zweiten Endbereich (24), der sich von dem zweiten distalen Ende des zentralen Bereichs (18) hin zum Querrand (12) erstreckt und zum Abdecken wenigstens eines Abschnitts des hinteren Damms einer Frau im Gebrauch ausgelegt ist; und
einen verdichteten Endkanal (31) in dem ersten oder dem zweiten Endbereich (22, 24),
**dadurch gekennzeichnet, daß** wenigstens zwei sich in Längsrichtung erstrekkende Gelenke (50, 52) innerhalb des Mittelbereichs (18) benachbart dem jeweiligen Längsrand (8, 10) angeordnet sind und der verdichtete Endkanal (31) in einem Abstand von wenigstens 3 mm von den Gelenken angeordnet ist, wobei jedes Gelenk (50, 52) eine sich in Längsrichtung erstreckende Vorzugsbiegeachse bereitstellen kann, wobei die Gelenke (50, 52) entlang wenigstens eines Abschnitts ihrer Länge in einem Abstand zueinander angeordnet sind, wobei die wenigstens zwei Gelenke jeweils einen Krümmungsradius aufweisen und ein Gelenk einen größeren Krümmungsradius als ein benachbartes Gelenk hat und wobei sich die Hygienedamenbinde bevorzugt längs des Gelenks knickt.

8. Hygienedamenbinde nach Anspruch 7, bei der ein erstes Gelenk einen größeren Krümmungsradius als ein zweites Gelenk aufweist und zur Längsmittellinie näher als das zweite Gelenke liegt, das einen kleineren Krümmungsradius als das erste Gelenk aufweist.

9. Hygienedamenbinde nach Anspruch 7, bei dem das erste Gelenk einen kleineren Krümmungsradius aufweist und näher an der Längsmittellinie liegt als das zweite Gelenk, das einen größeren Krümmungsradius als das erste Gelenk aufweist.

## Revendications

1. Serviette hygiénique destinée à être utilisée par une femme pour l'hygiène féminine et adaptée à être portée dans un sous-vêtement, comprenant une partie (6) de corps principal comportant une couche de couverture (2) pouvant revêtir le corps, perméable aux liquides, une couche d'arrêt (4) pouvant revêtir le vêtement, imperméable aux liquides, un élément absorbant (7) entre celles-ci, la partie (6) de corps principal comportant en outre des premier et deuxième bords longitudinaux (8, 10) opposés l'un à l'autre définissant une dimension en largeur, un axe médian longitudinal (11) parallèle aux bords longitudinaux (8, 10), des premier et deuxième bords transversaux (12, 14) opposés l'un à l'autre, définissant une dimension en longueur ;
une région centrale (18) comportant des première et deuxième extrémités distales (19, 20) opposées l'une à l'autre, définissant une longueur qui est suffisante pour recouvrir les grandes lèvres de la femme utilisant la serviette ;
une première région d'extrémité (22), s'étendant de la première extrémité distale (19) de la région centrale (18) au premier bord transversal (12) et étant adaptée à recouvrir au moins une partie du mont de Vénus du pubis de la femme utilisant la serviette ;
une deuxième région d'extrémité (24), s'étendant de la deuxième extrémité distale (19) de la région centrale (18) au deuxième bord transversal (14) et étant adaptée à recouvrir au moins une partie du périnée postérieur de la femme utilisant la serviette ;
un canal d'extrémité densifié (31) dans au moins l'une des première et deuxième régions d'extrémité (22, 24)
**caractérisé en ce que** au moins deux régions flexibles (50, 52) à extension longitudinale sont placées dans la région centrale (18), adjacentes à chaque bord longitudinal (8, 10), et **en ce que** ledit canal d'extrémité densifié est espacé desdites régions flexibles d'au moins 3 mm, chaque région flexible (50, 52) étant adaptée à donner un axe préférentiel de flexion à extension longitudinale, lesdites régions flexibles (50, 52) étant espacées l'une de l'autre sur au moins une partie de leur longueur, dans laquelle au moins une région flexible (50, 52) possède un rayon de courbure et une région flexible adjacente (50, 52) est rectiligne.

2. Serviette hygiénique selon la revendication 1, dans laquelle la première région flexible possède un rayon de courbure et est plus proche de l'axe médian longitudinal que la deuxième région flexible qui est rectiligne.

3. Serviette hygiénique selon la revendication 1, dans laquelle la première région flexible est sensiblement rectiligne et est plus proche de l'axe médian longitudinal que la deuxième région flexible qui possède un rayon de courbure.

4. Serviette hygiénique selon la revendication 1, dans laquelle la première région flexible est séparée de la deuxième région flexible sur une partie substantielle de leurs longueurs respectives, mais se terminent en association et sont en contact l'une avec l'autre à leurs extrémités distales respectives.

5. Serviette hygiénique selon la revendication 1, dans laquelle ledit canal d'extrémité densifié est espacé desdites régions flexibles d'au moins 5 mm.

6. Serviette hygiénique selon la revendication 5, dans laquelle ledit canal d'extrémité densifié est espacé desdites régions flexibles d'au moins 10 mm.

7. Serviette hygiénique destinée à être utilisée par une femme pour l'hygiène féminine et adaptée à être portée dans un sous-vêtement, comprenant une partie (6) de corps principal comportant une couche de couverture (2) pouvant revêtir le corps, perméable aux liquides, une couche d'arrêt (4) pouvant revêtir le vêtement, imperméable aux liquides, un élément absorbant (7) entre celles-ci, la partie (6) de corps principal comportant en outre des premier et deuxième bords longitudinaux (8, 10) opposés l'un à l'autre définissant une dimension en largeur, un axe médian longitudinal (11) parallèle aux bords longitudinaux (8, 10), des premier et deuxième bords transversaux (12, 14) opposés l'un à l'autre définissant une dimension en longueur ;
une région centrale (18) comportant des première et deuxième extrémités distales (19, 20) opposées l'une à l'autre, définissant une longueur qui est suffisante pour recouvrir les grandes lèvres de la femme utilisant la serviette ;
une première région d'extrémité (22), s'étendant de la première extrémité distale (19) de la région centrale (18) au premier bord transversal (12) et étant adaptée à recouvrir au moins une partie du mont de Vénus du pubis de la femme utilisant la serviette ;
une deuxième région d'extrémité (24), s'étendant de la deuxième extrémité distale (19) de la région centrale (18) au deuxième bord transversal (14) et étant adaptée à recouvrir au moins une partie du périnée postérieur de la femme utilisant la serviette ;
un canal d'extrémité densifié (31) dans au moins l'une des première et deuxième régions d'extrémité (22, 24)
**caractérisé en ce que** au moins deux régions flexibles (50, 52) à extension longitudinale sont placées dans la région centrale (18), adjacentes à chaque bord longitudinal (8, 10), et **en ce que** ledit canal d'extrémité densifié est espacé desdites régions flexibles d'au moins 3 mm, chaque région flexible (50, 52) étant adaptée à donner un axe préférentiel de flexion à extension longitudinale, lesdites régions flexibles (50, 52) étant espacées l'une de l'autre sur au moins une partie de leur longueur, dans laquelle au moins deux régions flexibles (50, 52) possèdent chacune un rayon de courbure et dans laquelle une région flexible possède un rayon de courbure supérieur à celui d'une région flexible adjacente, et dans lequel la serviette hygiénique s'incurve de préférence le long de la région flexible.

8. Serviette hygiénique selon la revendication 7, dans laquelle une première région flexible possède un rayon de courbure plus élevé par rapport à une deuxième région flexible et est plus proche de l'axe médian longitudinal que la deuxième région flexible qui possède un rayon de courbure plus faible par rapport à la première région flexible.

9. Serviette hygiénique selon la revendication 7, dans laquelle la première région flexible possède un rayon de courbure plus faible et est plus proche de l'axe médian longitudinal que la deuxième région flexible qui possède un rayon de courbure plus élevé par rapport à la première région flexible.
